# EUROPÄISCHE PATENTSCHRIFT

(11) **EP 0 804 494 B1**
(45) Veröffentlichungstag und Bekanntmachung des Hinweises auf die Patenterteilung: **26.07.2000**
(21) Anmeldenummer: 96901251.7
(22) Anmeldetag: 10.01.1996
(51) Int. Cl.: C08F 283/00, C08F 283/04, C08G 69/48

(54) **POLYMERISATIONSFÄHIGE DERIVATE VON POLYAMIDEN**
POLYMERISABLE DERIVATIVES OF POLYAMIDES
DERIVES POLYMERYSABLES DE POLYAMIDES

(30) Priorität: 20.01.1995 DE 19501726
(43) Veröffentlichungstag der Anmeldung: 05.11.1997
(73) Patentinhaber: MERCK PATENT GmbH, 64293 Darmstadt (DE)
(72) Erfinder: MÜLLER, Egbert, D-64390 Erzhausen (DE); GENSERT, Roland, D-64342 Seeheim-Oberbeerbach (DE); SEILER, Anja, D-64846 Gross-Zimmern (DE)
(86) Internationale Anmeldenummer: EP9600077
(87) Internationale Veröffentlichungsnummer: WO9622316

(56) Entgegenhaltungen:
- DE-A- 2 454 676
- US-A- 3 483 104
- US-A- 4 595 730
- DATABASE WPI Week 7538 Derwent Publications Ltd., London, GB; AN 75-63138w XP002003610 "modified polyamide compositions" & JP,A,50 072 992 (MATSUHITA) , 16.Juni 1975
- DATABASE WPI Week 7552 Derwent Publications Ltd., London, GB; AN 75-85563w XP002003611 "hardenable polyamide prepn." & JP,A,50 116 589 (MATSUHITA) , 11.September 1975
- CHEMICAL ABSTRACTS, vol. 118, no. 6, 8.Februar 1993 Columbus, Ohio, US; abstract no. 40505, KATO: "aromatic polyamide films treated..." XP002003609 & JP,A,04 164 934 (ASAHI) 10.Juni 1992

## Beschreibung

Die Erfindung betrifft polymerisationsfähige Derivate von Polyamiden, sowie Verfahren zur Pfropfpolymerisation auf derartig derivatisierten Polyamiden und schließlich nach diesen Verfahren hergestellte Pfropfpolymerisate und Formkörper.

Durch Pfropfungsreaktionen lassen sich die Oberflächen von Polymeren und von Formkörpern aus Polymeren verändern. Somit sind Eigenschaften erzielbar, die mit dem Basispolymeren allein nicht erreichbar sind.

Für die Ausführung von Pfropfungsreaktionen sind verschiedene Verfahren bekannt. Solche Verfahren mit ihren Vor- und Nachteilen sind beispielsweise in WO 91/03 506 eingehend diskutiert. So kann die Pfropfung mittels Anregung mit energiereicher Strahlung durchgeführt werden. Dabei treten jedoch unerwünschte Nebenreaktionen auf: Abbau der Polymerketten, welcher die mechanische Festigkeit der Polymeren beinträchtigt und Vernetzungsreaktionen, die zu einer Versprödung des Materials führen. Andere Verfahren der Pfropfpolymerisation, wie beispielsweise in DE 24 54 676 beschrieben, erfordern die Anwendung eines stark saurem Mediums, dabei werden Polyamide jedoch teilweise hydrolytisch gespalten. Wieder andere Verfahren der Pfropfpolymerisation beruhen auf der Kettenübertragung, indem durch einen Radikalinitiator eine Homopolymerisation des Monomeren in Gegenwart des zu pfropfenden Basispolymeren induziert wird. Die Pfropfstelle läßt sich nicht gezielt beeinflussen. In WO 91/03 506 wird ein Verfahren zur Pfropfung auf Polyamidmembranen beschrieben, bei dem die Radikalreaktion über eine Behandlung mit Tetrachlorkohlenstoff in Gegenwart eines Reduktionsmittels gestartet wird. Bei diesem Verfahren wird die Radikalkette durch einen nicht bekannten Reaktionsmechanismus ausgehend von einem Halogenamid gestartet. Aus der Literatur ist bekannt, daß Halogenamide sehr reaktiv sind. Dies kann zu unerwünschten Reaktionen führen, wie der kovalenten Bindung von Proteinen bei der Benutzung der derivatisierten Membran. Bei der Verwendung von Reduktionsmitteln werden außerdem die mechanischen Eigenschaften des Polyamides beeinträchtigt.

JP 50 072 992 beschreibt die Reaktion von gemischten Polyamiden mit Verbindungen, die reaktive Doppelbindungen oder Epoxygruppen enthalten. JP 50 116 589 offenbart die Reaktion von Polyamiden mit Glycidylestern von ungesättigten Carbonsäuren. In US 4 595 730 wird ein Verfahren beschrieben, um ethylenisch ungesättigte Polyamide in der Schmelze direkt aus Vorläufer-Monomeren des Polyamids und ungesättigten Verbindungen herzustellen, die mit den terminalen Amino-oder Carboxyl-Gruppen des entstehenden Polyamids reagieren können.

Aufgabe der Erfindung ist es, ein Verfahren zur gezielten Derivatisierung von Polyamiden bereitzustellen. Diese derivatisierten Polymere können anschließend nach bekannten Verfahren weiter umgesetzt werden. Dabei sollen derivatisierte Polyamide bereitgestellt werden, deren mechanischen Eigenschaften soweit erhalten sind, daß diese Verfahren beispielsweise auch für Hohlfasermembranen anwendbar sind.

Gegenstand der Erfindung sind daher polymere Sorbentien, durch die folgenden Reaktionsschritte erhältlich sind:
a) Einführung von polymerisierbaren Doppelbindungen durch Umsetzung des Polyamides mit einer Verbindung, die sowohl eine polymerisierbare Doppelbindung als auch einen Oxiranring enthält;
b) Polymerisation von Monomeren auf das nach Schritt a) derivatisierte Polyamid, wobei die genannten Monomeren eine Epoxidgruppe, eine Azlactongruppe oder eine Vorläufergruppe für eine Azlactongruppe oder einen Separationseffektor enthält;
c) optionale Umsetzung der genannten Epoxid- oder Azlacton-gruppe in einen Separationseffektor.

Bevorzugte Ausführungsform sind polymere Sorbentien, bei denen in Schritt a) als Verbindung, die sowohl eine polymerisierbare Doppelbindung als auch einen Oxiranring enthält, eine Verbindung der Formel I verwendet wird:

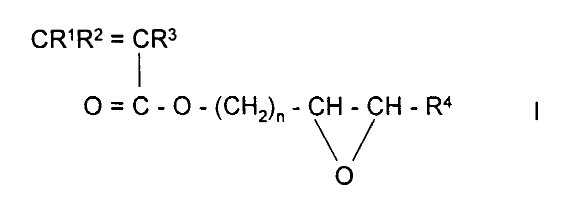

Darin bedeuten
- R¹, R² und R³: unabhängig voneinander H oder CH₃,
- R⁴: H, Alkyl mit 1-5 C-Atomen oder Aryl mit 6-12 C-Atomen
und
- n: eine ganzen Zahl zwischen 1 und 5.

Eine weitere bervorzugte Ausführungsform sind polymere Sorbentien, bei deren Herstellung in Schritt b) Acrylsäure verwendet wird.

Gegenstand der Erfindung sind auch polymere Sorbentien, in denen das aufpolymerisierte Polymer Monomereinheiten Formel II, V, III oder IV enthält:

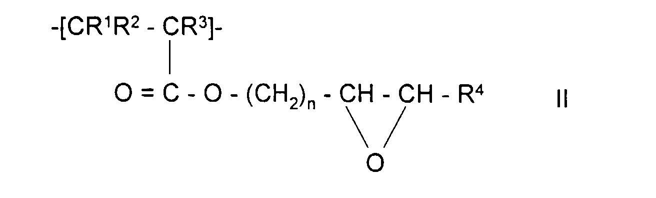

In Formel II bedeuten:
- R¹, R² und R³: unabhängig voneinander H oder CH₃,
- R⁴: H, Alkyl mit 1-5 C-Atomen oder Aryl mit 6-12 C-Atomen
und
- n: eine ganzen Zahl zwischen 1 und 5.

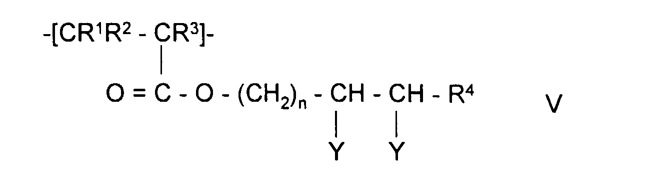

In Formel V bedeuten:
- R¹, R² und R³: unabhängig voneinander H oder CH₃,
- R⁴: H, Alkyl mit 1-5 C-Atomen oder Aryl mit 6-12 C-Atomen
- n: eine ganzen Zahl zwischen 1 und 5,
ein Rest Y einen Rest der Formel VI und der andere Rest Y OH 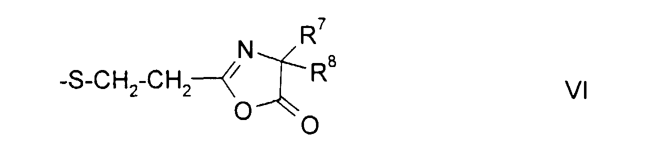 und
- R⁷ und R⁸: unabhängig voneinander

H oder Alkyl mit 1-5 C-Atomen.

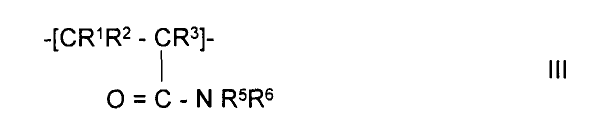

In Formel III bedeuten:
- R¹, R² und R³: unabhängig voneinander H oder CH₃,
- R⁵: H, mit -COOH, mit -SO₃H oder mit -NR⁷R⁸ substituiertes Alkyl mit 1-5 C-Atomen oder mit -COOH, -SO₃H oder mit -NR⁷R⁸ substituiertes Aryl mit 6-12 C-Atomen,
- R⁶: mit -COOH, mit -SO₃H oder mit -NR⁷R⁸ substituiertes Alkyl mit 1-5 C-Atomen oder mit -COOH, -SO₃H oder mit - NR⁷R⁸ substituiertes Aryl mit 6-12 C-Atomen,
wobei
- R⁵ und R⁶: so abgestimmt sind, daß entweder beide Reste sauer oder basisch oder einer der Reste neutral ist,
und
- R⁷ und R⁸: unabhängig voneinander H, Alkyl mit 1-5 C-Atomen.

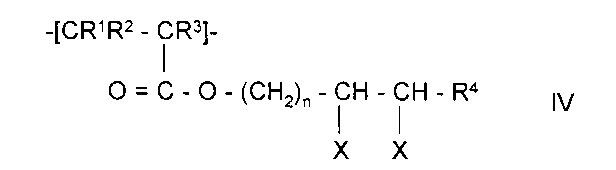

In Formel IV bedeuten:
- R¹, R² und R³: unabhängig voneinander H oder CH₃,
- R⁴: H, Alkyl mit 1-5 C-Atomen oder Aryl mit 6-12 C-Atomen
- n: eine ganzen Zahl zwischen 1 und 5,
ein Rest X einen Separationseffektor, sowie der andere Rest X OH.

Bevorzugte Ausführungsform eines polymeren Sorbens, bei dem das aufpolymerisierte Polymer Monomereinheiten entsprechend Formel IV besitzt, ist ein Sorbens, dessen Separationseffektor insbesondere eine der folgenden Bedeutungen aufweist:
a) eine ionische Gruppe, ausgewählt aus -PO₄H₂, SO₃H, -NR⁷R⁸ oder -N⁺R⁷R⁸R⁹, worin
   - R⁷ und R⁸: unabhängig voneinander H, Alkyl mit 1-5 C-Atomen
   und
   - R⁹: Alkyl mit 1-5 C-Atomen
   mit der Maßgabe, daß wenn X = -N⁺R⁷R⁸R⁹, R⁷ und R⁸ nicht H sein können,
b) eine hydrophobe Gruppierung -OR¹⁰ oder -NHR¹⁰, wobei
   - R¹⁰: C₁-C₂₀-Alkyl, C₆-C₂₅-Aryl, C₇-C₂₅-Alkylaryl oder C₇-C₂₅-Arylalkyl bedeuten, und wobei diese Reste auch mit Nitril oder C₁-C₅-Alkoxy derivatisiert sein können, und wobei auch eine oder mehrere nicht benachbarte CH₂-Gruppen durch NH oder O oder auch eine oder mehrere CH Gruppen durch N ersetzt sein können;
c) eine Metallchelat-Gruppierung;
d) ein thiophiler Rest.

Thiophile Reste sind beispielsweise in EP 0 165 912 offenbart.

Insbesondere die polymeren Sorbentien, die Monomereinheiten der Formel II oder V enthalten, können für die Herstellung von Affinitätsträgern oder zur Immobilisierung von Enzymen verwendet werden.

Gegenstand der Erfindung sind somit auch Affinitätsträger und immobilisierte Enzyme herstellbar aus einem erfindungsgemäßen polymeren Sorbens.

Es sind poröse und unporöse Formkörper aus Polyamiden bekannt; dazu gehören beispielsweise Membranen, Schwämme, Schläuche und Hohlfasermembranen. Gegenstand der Erfindung sind somit auch derartige Formkörper, die im wesentlichen aus einem erfindungsgemäßen polymeren Sorbens bestehen, und die zusätzlich auch Affinitätsliganden oder immobilierte Enzyme enthalten können.

Ferner ist Gegenstand der Erfindung ein Verfahren zur chromatographischen Stofftrennung, bei dem ein erfindungsgemäßes polymeres Sorbens verwendet wird.

Erfindungsgemäß werden bei dem ersten Reaktionsschritt ungesättigte Reste in das Polyamid eingeführt. Geeignetete Polyamide sind dem Fachmann bekannt und sind auch kommerziell erhältlich. Dazu gehören z.B. die unter dem Handelsnamen NYLON® bekannten Polymere, z.B. NYLON® 66. Poröse oder unporöse Formkörper bestehend aus derartigen Polyamiden sind ebenfalls bekannt und auch kommerziell erhältlich; dazu gehören beispielsweise perlförmige Formkörper, Membranen, Schläuche, Hohlfasermembranen, Schwämme. Das Polyamid, das derivatisiert werden soll, wird mit einer Verbindung, die neben einer ungesättigten C=C-Gruppe auch einen Oxiranring enthält, umgesetzt. Es wurde gefunden, daß diese Umsetzung in hervorragender Weise ohne den Zusatz von Halogenkohlenwasserstoffen oder Reduktionsmitteln möglich ist. Die Umsetzung erfolgt in wäßrig-organischer Lösung bei einem pH > 5. Als organische Lösungsmittel werden insbesondere Dimethylformamid (DMF), Dimethylsulfoxid (DMSO) und Dioxan bevorzugt. Dabei beträgt der Anteil des organischen Lösungsmittels typischerweise mehr als 30 Gewichtsprozent. Es ist auch möglich, die Reaktion in reinem organischen Lösungsmittel auszuführen. Wird die Reaktion bei einem pH > 10 ausgeführt, so kann auch in rein wäßriger Phase gearbeitet werden. Bei einer zu derivatisierenden Oberfläche von 100 cm² werden typischerweise 1 bis 20 g einer Verbindung, die neben einer ungesättigten C=C-Gruppe auch einen Oxiranring enthält, beispielsweise Glycidylmethacrylat, gelöst in 50 bis 500 ml Lösungsmittel, umgesetzt. Die Reaktion wird typischerweise bei 30 - 60 °C ausgeführt und dauert typischerweise zwischen 30 Minuten und einigen Stunden. Für die Einstellung des pH kann in wasserhaltigen Medien ein Puffer zugesetzt werden; typischerweise beträgt die Konzentration des Puffers zwischen 10 mM und 2 M. Geeignete Puffer sind dem Fachmann bekannt; dazu gehören beispielsweise Borat- und Carbonatpuffer. Die Umsetzung in rein wäßriger Lösung kann auch in verdünnter Alkalihydroxidlösung (z.B. 0,1 bis 2 M Natrium- oder Kaliumhydroxidlösung) erfolgen. Zusätze eines halogenierten Kohlenwasserstoffes oder eines Reduktionsmittels ist nicht notwendig und erfindungsgemäß nicht vorgesehen.

Verbindungen, die neben einer ungesättigten C=C-Gruppe auch einen Oxiranring enthalten, sind dem Fachmann bekannt; dazu gehören insbesondere Glycidylmethacrylat, Glycidylacrylat, Vinylglycidylether und auch Vinylglycidylurethan. Glycidylmethacrylat wird bevorzugt verwendet.

Durch die Umsetzung des Polyamids mit einer Verbindung, die neben einer ungesättigten C=C-Gruppe auch einen Oxiranring enthält, werden auf sehr schonende Weise ungesättigte C=C-Gruppen in das Polyamid eingeführt. An diese Gruppen können weitere Monomere nach allgemein bekannten Verfahren polymerisiert werden. Die Auswahl dieser Monomeren richtet sich nach dem vorgesehenen Verwendungszweck der derivatisierten Membran:
a) Aus DE 38 11 042 sind unter anderem Monomere bekannt, die zur Herstellung von lonenaustauschem geeignet sind; dazu gehören beispielsweise Acrylsäure, N-(Sulfoethyl)-acrylamid, 2-Acrylamido-2-methylpropansulfonsäure, N,N-Dimethylaminoethyl-acrylamid, N,N-Diethylaminoethyl-acrylamid, sowie Trimethylammoniumethyl-acrylamid.
   Andere in dieser Druckschrift genannte Monomere erlauben die Bindung von Affinitätsliganden oder von Enzymen, oder eignen sich für reversed phase Chromatographie: dazu gehören beispielsweise Acrylsäure, Acrylamid, Allylamin oder Acrylnitril.
b) Aus DE 43 10 964 sind Monomere bekannt, die einen Oxiranring, einen Azlactonring oder eine Gruppierung enthalten, die in einen Azlactonring umgesetzt werden kann. Polymere, die derartige Monomere enthalten, sind besonders gut für die Bindung von Affinitätsliganden oder von Enzymen geeignet. Affinitätsliganden sind beispielhaft in DE 43 10 964 offenbart.
   Weiterhin können die Epoxidgruppen in derartigen Polymeren in vorteilhafter Weise weiter umgesetzt werden, wodurch lonenaustauscher, thiophile Sorbentien oder Sorbentien für die Metallchelat- oder die hydrophobe Chromatographie bereitgestellt werden. Dabei werden beispielsweise Phosphorsäure, Diethylamin, Trimethylamin, schweflige Säure oder auch Komplexbildner wie Iminodiessigsäure an den Oxiranring addiert.
   Die Herstellung von thiophilen Sorbentien und von Sorbenzien für die Metallchelatchromatographie ist in DE 43 10 964 offenbart.
   In DE 43 33 674 und in DE 43 33 821 sind derartige Umsetzungen, mit derer Hilfe lonenaustauscher bereitgestellt werden können, offenbart.
   In DE 43 23 913 werden Sorbenzien für die hydrophobe Interaktionschromatographie beschrieben.

Erfindungsgemäß werden die Gruppen, die nach den oben genannten Verfahren in den chromatographischen Träger eingeführt werden, und die für die Trennung der Analyte wesentlich sind, zusammenfassend als Separationseffektoren bezeichnet.

Einzelheiten der Herstellung der verschiedenen Sorbenzien und deren Verwendung können den oben genannten Druckschriften entnommen werden; die diesbezügliche Offenbarung dieser Druckschriften ist durch Bezugnahme in die vorliegende Anmeldung eingeführt.

Die verschiedenen Gruppierungen, die die Trennung der Analyte bewerkstelligen, werden erfindungsgemäß als Separationseffektoren zusammengefaßt. Beispiele können den obigen Druckschriften entnommen werden.

Die Reaktion, bei der weitere Monomere auf das erfindungsgemäß derivatisierte Polyamid aufpolymerisiert werden, kann unter Rühren, durch Um- oder Durchpumpen der Reaktionslösung erfolgen. Beim Umpumpen wird die Reaktionslösung nach dem Durchströmen der Polyamidmembran wieder zurückgeführt und erneut durchgepumpt, beim Durchpumpen wird die Reaktionslösung nach dem Durchströmen der Polyamidmembran verworfen. Die letztgenannte Verfahrensvariante ist bevorzugt.

Die erfindungsgemäß derivatisierten und gepfropften Membranen, die Separationseffektoren enthalten, können für Stofftrennungen in ähnlicher Weise eingesetzt werden, wie es beispielsweise für partikuläre Sorbenzien mit ähnlichen Separationseffektoren üblich ist. Wegen der kurzen Verweilzeiten am Sorbens, ist dabei die Gefahr, daß insbesondere die biologische Aktivität verringert wird, verringert. Die Isolierung von natürlichen oder rekombinanten Enzymen aus Rohextrakten ist für derartige Anwendungen ein Beispiel. So umfaßt die Isolierung beispielsweise von Glucosedehydrogenase aus Bacillus megaterium oder von Proteinase K aus Tritirachium album einen Chromatographieschritt an einem Sorbens mit ternären Aminogruppen (z.B. DEAE) oder mit quatemären Ammoniumgruppen (z.B. TMAE). Für die Isolierung von polyklonalen oder monoklonalen Immunglobulinen wie IgG oder IgM werden in Abhängigkeit vom isoelektrischen Punkt des Immunglobulins Anionen- oder Kationenaustauscher (z.B. mit TMAE- oder SO₃⁻-Gruppen), insbesondere aber auch thiophile oder hydrophobe Träger verwendet. Der Erhalt der biologischen Aktivität ist insbesondere bei der Isolierung von Gerinnungsfaktoren oder anderer Wirkstoffe aus Blutplasma oder -serum von entscheidender Wichtigkeit. So wird bei der Isolierung des Prothrombinkomplexes oder von α₁-Antitrypsin an einem DEAE-Träger chromatographiert. Für die Anreicherung von Faktor IX ist die Chromatographie an einem Träger mit Aminogruppen üblich. Bei der Isolierung von Faktor VIII sind Chromatographieschritte an einem Sorbens mit ternären Aminogruppen (z.B. DEAE) oder mit quaternären Ammoniumgruppen (z.B. TMAE) oder auch an hydrophoben Trennmaterialien üblich.

Weitere Beispiele von Stofftrennungen, bei denen der Erhalt der biologischen Aktivität besonders wichtig ist, sind dem Fachmann aus der Literatur bekannt. In allen diesen Fällen kann ein partikulärer chromatographischer Träger mit einem bestimmten Separationseffektor durch eine erfindungsgemäße Membran ersetzt werden, die denselben oder einen ähnlichen Separationseffektor enthält. Beispiele für Membranen, die derartige Separationseffektoren enthalten, sind bereits genannt und sind insbesondere in den folgenden Beispielen offenbart.

Die folgenden Beispiele sollen den Gegenstand der Erfindung näher erläutern; sie stellen keine Einschränkung des Erfindungsgegenstandes dar.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen, sowie der korrespondierenden Anmeldung DE 1 95 01 726.9, eingereicht am 20. Januar 1995, sind durch Bezugnahme in diese Anmeldung eingeführt.

### Beispiele

Im folgenden wird unter Raumtemperatur eine Temperatur zwischen 15 und 30 °C verstanden.

### Beispiel 1: Einführung von C=C-Bindungen in ein Polyamid in wäßriger Lösung (Variante A)

Zur Durchführung der Synthese wird ein Polyamidfaserbündel aus NYLON (Faserbündel aus 64 Fasern, 32 cm lang, etwa 200 um innerer Durchmesser, etwa 2 mm äußerer Durchmesser, mittlerer Porendurchmesser 1 - 2 µm, Oberfläche 97 cm²) auf 30 cm gekürzt und in eine 300-10 mm Chromatographiesäule SUPERFORMANCE® (Fa. E. Merck) gepackt. An diese Säule wird eine inerte Pumpe angeschlossen. Die Apparatur wird zunächst mit Wasser gespült. Für die Umsetzung werden 20 g Glycidylmethacrylat in 200 ml 1 M NaOH gelöst und mit hoher Geschwindigkeit (5 ml/min) bei 40 °C zwei Stunden im Kreis gepumpt. Anschließend wird die derivatisierte Membran mit Wasser gespült.

### Beispiel 2: Einführung von C=C-Bindungen in ein Polyamid (wäßrig - organische Lösung; Variante B)

10 g Glycidylmethacrylat werden in 200 ml wäßrigem Dimethylformamid (50 Volumen-%) gelöst und in der in Beispiel 1 beschriebenen Apparatur umgepumpt (zwei Stunden bei 60 °C). Anschließend wird die derivatisierte Hohlfasermembran mit Wasser gespült.

### Beispiel 3: Einführung von C=C-Bindungen in ein Polyamid (wäßrig - organische Lösung; Variante C)

10 g Glycidylmethacrylat werden in 200 ml wäßrigem Dimethylsulfoxid (50 Volumen-%) gelöst und in der in Beispiel 1 beschriebenen Apparatur umgepumpt (zwei Stunden bei 60 °C). Anschließend wird die derivatisierte Hohlfasermembran mit Wasser gespült.

### Beispiel 4: Einführung von C=C-Bindungen in ein Polyamid (wäßrig - organische Lösung; Variante D)

10 g Glycidylmethacrylat werden in 200 ml wäßrigem Dimethylformamid (50 Volumen-% in 0,5 M Natriumcarbonatpuffer, pH 10) gelöst und in der in Beispiel 1 beschriebenen Apparatur umgepumpt (zwei Stunden bei 60 °C). Anschließend wird die derivatisierte Hohlfasermembran mit Wasser gespült.

### Beispiel 5: Einführung von C=C-Bindungen in ein Polyamid (wäßrig - organische Lösung; Variante E)

10 g Glycidylmethacrylat werden in 200 ml wäßrigem Dimethylsulfoxid (50 Volumen-% in 0,5 M Natriumcarbonatpuffer, pH 10) gelöst und in der in Beispiel 1 beschriebenen Apparatur.umgepumpt (zwei Stunden bei 60 °C). Anschließend wird die derivatisierte Hohlfasermembran mit Wasser gespült.

### Beispiel 6: Einführung von C=C-Bindungen in ein Polyamid (wäßrig - organische Lösung; Variante F)

10 g Glycidylmethacrylat werden in einer Mischung aus 40 ml Dioxan und 160 ml Wasser gelöst und anschließend 17,5 g NaOH-Losöung (32 Gew.-%) zugefügt. Diese Lösung wird anschließend in der in Beispiel 1 beschriebenen Apparatur umgepumpt (Flußgeschwindigkeit 3 ml/min.; eine Stunde bei 50 °C). Anschließend wird die derivatisierte Hohlfasermembran mit 100 ml Wasser, 200 ml Aceton und 100 ml Toluol gespült.

### Beispiel 7: Einführung von C=C-Bindungen in eine Flachmembran aus Polyamid

Drei Lagen einer Flachmembran aus Polyamid (jeweils 0,3 mm dick; 12 cm Durchmesser; 0,2 µm Porenweite) wird in einen handelsüblichen Membranhalter eingespannt und entsprechend von Beispiel 6 mit Glycidylmethacrylat umgesetzt. Anschließend wird die derivatisierte Membran mit Wasser gespült.

### Beispiel 8: Pfropfpolymerisation mit Glycidylmethacrylat

Die nach Beispiel 1 derivatisierte Hohlfasermembran wird in der dort beschriebenen Apparatur mit zunächst mit Aceton, dann mit Toluol (jeweils 200 ml) gespült. Anschließend wird eine Lösung von 20 g Glycidylmethacrylat und 1 g Azoisobutyronitril (Polymerisationsinitiator) in 200 ml Toluol bei 80 °C eine Stunde-umgepumpt. Die derivatisierte Hohlfasermembran wird anschließend mit Toluol und Aceton gespült.

### Beispiel 9: Pfropfpolymerisation mit Glycidylmethacrylat

Durch die nach Beispiel 6 derivatisierte und gespülte Hohlfasermembran wird in der in Beispiel 1 beschriebenen Apparatur wird eine Lösung von 15 g Glycidylmethacrylat und 1 g Azoisobutyronitril (Polymerisationsinitiator) in 200 ml Toluol bei 100 °C eine Stunde umgepumpt (7 ml/min). Die derivatisierte Hohlfasermembran wird anschließend mit Toluol und Aceton gespült.

### Beispiel 10: Propfpolymerisation mit Acrylsäure

Die nach Beispiel 1 derivatisierte Hohlfasermembran wird in der dort beschriebenen Apparatur mit zunächst mit Aceton, dann mit Toluol (jeweils 200 ml) gespült. Anschließend wird eine Lösung von 10 g Acrylsäure und 1 g Azoisobutyronitril in 200 ml Toluol bei 80°C eine Stunde umgepumpt. Die derivatisierte Hohlfasermembran wird anschließend mit Toluol, Aceton sowie Wasser und 1 M NaOH gewaschen.

### Beispiel 11: Pfropfpolymerisation mit Glycidylmethacrylat im Durchfluss

Die nach Beispiel 6 derivatisierte Hohlfasermembran wird in der dort beschriebenen Apparatur mit zunächst mit Aceton, dann mit Toluol (jeweils 200 ml) gespült. Es wird eine Lösung von 20 g Glycidylmethacrylat und 1 g Azoisobutyronitril (Polymerisationsinitiator) in 200 ml Toluol hergestellt. Diese Lösung wird mit einem linearen Fluß von 10 cm/min bei 90 °C durch die Vorrichtung gepumpt; dieser Vorgang dauert ca. 20 Minuten. Die derivatisierte Hohlfasermembran wird anschließend mit Toluol und Aceton gespült.

### Beispiel 12: Pfropfpolymerisation mit Glycidylmethacrylat im batch

10 g Polyamidpulver (mittlere Partikelgröße 200 µm, mittlere Porengröße 3 um) werden entsprechend Beispiel 1 derivatisiert. Anschließend wird das derivatisierte Polyamidpulver in einer Reaktionslösung aus 20 g Glycidylmethacrylat und 1 g Azoisobutyronitril (Polymerisationsinitiator) in 200 ml Toluol in einem Dreihalskolben unter Rühren bei 85 °C drei Stunden umgesetzt und das Reaktionsprodukt anschließend mit Toluol und Aceton gespült.

### Beispiel 13: Pfropfpolymerisation an einer derivatisierten Flachmembran mit Glycidylmethacrylat im Durchfluss

Die nach Beispiel 7 derivatisierten Polyamidflachmembranen werden entsprechend Beispiel 10 im Durchfluß mit einer Reaktionslösung aus 200 g Glycidylmethacrylat und 10 g Azoisobutyronitril (Polymerisationsinitiator) in 2 l Toluol bei 95 °C umgesetzt. Die derivatisierte Membran wird anschließend mit Toluol und Aceton gespült.

### Beispiel 14: Pfropfpolymerisation an einer derivatisierten Flachmembran mit Glycidylmethacrylat im batch

Die nach Beispiel 7 derivatisierten Polyamidflachmembranen werden auf ein Drahtgestell aufgewickelt und in einer Reaktionslösung aus 200 g Glycidylmethacrylat und 10 g Azoisobutyronitril (Polymerisationsinitiator) in 2 l Toluol bei 85 °C unter Rühren umgesetzt (1 Stunde). Die derivatisierte Membran wird anschließend mit Toluol und Aceton gespült.

### Beispiel 15: Bestimmung der Epoxygruppendichte

Die Epoxygruppendichte der nach den Beispielen 8, 11, 12, 13 und 14 derivatisierten Membranen wird durch Perchlorsäuretitration im wasserfreiem Medium bestimmt (M.Pribl; Fresenius Z. Anal. Chem. **303**, 113-116; 1980)). Dazu wird die nach den Beispielen derivatisierte Hohlfasermembran in kleine Stücke geschnitten. Etwa 1 g der Membranstückchen werden titriert.

| Es wurden gefunden (mmol Epoxygruppen / g Membran): | | | | | |
|---|---|---|---|---|---|
| Beispiel | 8 | 11 | 12 | 13 | 14 |
| mmol/g | 1,0 | 1,5 | 0,3 | 0,5 | 0,13 |

### Beispiel 16: Modifizierung einer Membran zu einem schwachbasischen Anionenaustauscher

Die nach Beispiel 6 derivatisierte Membran wird in der in Beispiel 1 beschriebenen Apparatur zunächst mit Wasser gespült. Anschließend werden 200 ml einer wäßrige Diethylaminlösung (50 Volumen-%) 6 Stunden bei Raumtemperatur durch die Apparatur gepumpt. Schließlich wird die erhaltene lonenaustauschmembran mit 0,5 M Natriumphosphatpuffer (pH 7) gewaschen, bis das Eluat neutral ist.

### Beispiel 17: Modifizierung einer Membran zu einem mit Azlactongruppen aktivierten Träger

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. Eine Lösung von 12,4 g Natriumcarbonat-Monohydrat in 200 ml Wasser wird mit konzentrierter HCI auf pH 11 eingestellt und 30 g Natriumhydrogensulfid-Hydrat in dieser Lösung gelöst. Die Lösung wird auf 40 °C erwärmt und eine Stunde mit einem Fluß von 7 ml/min durch die auf 40 °C temperierte Säule gepumpt. Anschließend wird die Säule mit 100 ml Wasser und 200 ml Aceton gespült und im Vakuumtrockenschrank bei 50 °C über Nacht getrocknet.

40 g Vinyldimethylazlacton werden in 200 ml Dimethylformamid gelöst und 3,3 g 1,8-Diazabicylclo(5,4,0)undecen-7-en zugefügt. Diese Lösung wird bei Raumtemperatur 8 Stunden bei einem Fluß von 7 ml/min durch die Vorrichtung gepumpt. Anschließend wird die Reaktion ohne umzupumpen über Nacht fortgeführt. Die Säule wird mit 100 ml Dimethylformamid gespült und bei 80 °C für zwei Stunden mit Ethylacetat extrahiert.

### Beispiel 18: Modifizierung einer Membran zu einem Träger mit Phenolethergruppen (für die hydrophobe Interaktionschromatographie)

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. 10 g Phenol werden in 200 ml Wasser zusammen mit 24 g Natronlauge (32 Gew.-%) gelöst und bei 90 °C und einem Fluß von 5 ml/min 6 Stunden durch die Apparatur gepumpt. Die Reaktion wird ohne umzupumpen über Nacht bei Raumtemperatur fortgeführt. Anschließend wird mit Wasser, 1 M Natronlauge, Wasser, 0,1 M Phosphatpuffer pH 7 , Wasser und Aceton gespült. Die Membranen werden anschließend bei Raumtemperatur im Vakuumtrockenschrank über Nacht getrocknet.

### Beispiel 19: Modifizierung einer Membran zu einem thiophilen Affinitätsträger

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. 7,75 g Natriumcarbonat-Monohydrat werden in 125 ml Wasser gelöst und anschließend 18,75 g Natriumhydrogensulfid-Hydrat zugefügt. Die Lösung wird auf 40 °C erwärmt und bei einem Fluß von 3 ml/min eine Stunde durch die Apparatur gepumpt. Anschließend wird mit 100 ml Wasser gespült.

7,75 g Natriumcarbonat-Monohydrat werden in 125 ml Wasser gelöst und 5 ml Divinylsulfon zugefügt. Die Lösung wird auf 40 °C erwärmt und bei einem Fluß von 3 ml/min eine Stunde durch die Apparatur gepumpt. Anschließend wird mit 100 ml Wasser gespült.

7,75 g Natriumcarbonat-Monohydrat werden in 125 ml Wasser gelöst und 20 ml Mercaptoethanol zugefügt. Die Lösung wird auf 40 °C erwärmt und bei einem Fluß von 2,5 ml/min eine Stunde durch die Apparatur gepumpt. Anschließend wird mit je 100 ml Wasser, 1 M Natronlauge, Wasser, 1 M HCI, Wasser, 0,1 M Phosphatpuffer (pH 7) und Wasser, gespült. Zuletzt wird mit 200 ml wäßrigem Ethanol (20 Gew.-%) gespült.

### Beispiel 20: Modifizierung einer Membran zu einem starkbasischen Anionenaustauscher

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. 100 ml wäßrige Trimethylaminlösung (45 Gew.-%; Kat.-Nr. 821 177; Fa. Merck, Darmstadt) werden mit 100 ml Wasser verdünnt und bei Raumtemperatur drei Stunden mit 5 ml/min durch die Apparatur gepumpt; anschließend wird die Reaktion ohne umzupumpen über Nacht weitergeführt. Die Membran wird mit Wasser, 1 M Natronlauge, Wasser, 0,1 M Phosphatpuffer (pH 7), Wasser und Aceton gespült und im Vakuumtrockenschrank bei Raumtemperatur über Nacht getrocknet.

### Beispiel 21: Modifizierung einer Membran zu einem Träger mit Aminogruppen

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. Wäßrige Ammoniaklösung (ca. 100 ml; 32 Gew.-%) werden in die auf 30 °C erwärmte Apparatur eingefüllt. Während des Verlaufes von drei Stunden wird Ammoniaklösung zugefügt, so daß die Apparatur vollständig gefüllt bleibt. Anschließend wird der Auslauf der Apparatur verschlossen und die Reaktion über Nacht bei Raumtemperatur weitergeführt. Die Membran wird mit Wasser, 1 M Natronlauge, Wasser, 0,1 M Phosphatpuffer (pH 7), Wasser und Aceton gespült und im Vakuumtrockenschrank bei Raumtemperatur über Nacht getrocknet.

### Beispiel 22: Modifizierung einer Membran zu einem Kationenaustauscher

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. 10 g Natriumdihydrogenphosphat, 40 g Natriumsulfit und 10 g Tetrabutylammoniumhydrogensulfat werden in 200 ml Wasser gelöst und auf pH 8 eingestellt. Diese Lösung wird auf 95 °C erwärmt und 2,5 Stunden mit 7 ml/min durch die Apparatur gepumpt. Die Membran wird mit Wasser, 1 M Natronlauge, Wasser, 0,1 M Phosphatpuffer (pH 7), Wasser und Aceton gespült und im Vakuumtrockenschrank bei Raumtemperatur über Nacht getrocknet.

### Beispiel 23: Modifizierung einer Membran zu einem Träger für die Metallchelat-Affinitätschromatographie

Die nach Beispiel 6 derivatisierte Membran und nach Beispiel 9 mit Epoxypropylmethacrylat gepfropfte Membran wird in der in Beispiel 1 beschriebenen Apparatur zunächst mit Wasser gespült. 26 g Iminodiessigsäure werden in 200 ml Wasser gelöst und mit Natronlauge auf pH 11 eingestellt. Diese Lösung wird auf 60 °C erwärmt und drei Stunden mit 5 ml/min durch die Apparatur gepumpt; anschließend wird die Reaktion ohne umzupumpen über Nacht bei Raumtemperatur weitergeführt. Die Membran wird mit Wasser, 1 M Natronlauge, Wasser, 0,1 M Phosphatpuffer (pH 7), Wasser und Aceton gespült und im Vakuumtrockenschrank bei Raumtemperatur über Nacht getrocknet.

### Beispiel 24: Bestimmung der Proteinbindungskapazität

0,5 g der nach Beispiel 16 modifizierten und in kleine Stücke geschnittenen Membran werden in 10 ml einer Rinderserumalbuminlösung (5 mg/ml in 20 mM Tris pH 8,2) 3 Stunden geschüttelt. Die Membranstücke werden abgesaugt. Der Überstand wird verworfen. Das gebundene Protein wird durch Behandlung mit 20 ml einer Pufferlösung aus 20 mM Tris + 1 M Kochsalz pH 8 desorbiert. Der Gehalt wird photometrisch bei 280 nm bestimmt.

Die gefundene Proteinbindungkapazität beträgt 44 mg Protein/g Membran.

## Patentansprüche

1. Polymeres Sorbens erhältlich durch folgende Reaktionsschritte:
a) Einführung von polymerisierbaren Doppelbindungen durch Umsetzung eines Polyamides mit einer Verbindung, die sowohl eine polymerisierbare Doppelbindung als auch einen Oxiranring enthält;
b) Polymerisation von Monomeren auf das nach Schritt a) derivatisierte Polyamid, wobei die genannten Monomeren eine Epoxidgruppe, eine Azlactongruppe oder eine Vorläufergruppe für eine Azlactongruppe oder einen Separationseffektor enthält;
c) optionale Umsetzung der genannten Epoxid- oder Azlacton-gruppe in einen Separationseffektor.

2. Polymeres Sorbens nach Anspruch 1, wobei in Schritt a) als Verbindung, die sowohl eine polymerisierbare Doppelbindung als auch einen Oxiranring enthält, eine Verbindung der Formel I verwendet wird, 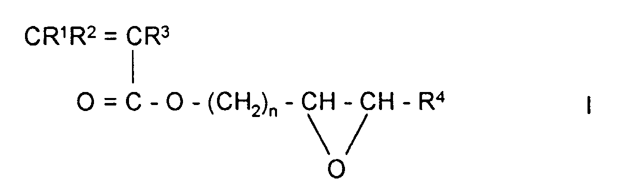 worin
R¹, R² und R³ unabhängig voneinander H oder CH₃,
R⁴ H, Alkyl mit 1-5 C-Atomen oder Aryl mit 6-12 C-Atomen
und
n eine ganzen Zahl zwischen 1 und 5
bedeuten.

3. Polymeres Sorbens nach Anspruch 1 oder 2, dadurch gekennzeichnet, daß als Monomer in Reaktionsschritt b) Acrylsäure verwendet wird.

4. Polymeres Sorbens nach Anspruch 1 oder 2, dadurch gekennzeichnet, daß das aufpolymerisierte Polymer Monomereinheiten der Formel II, 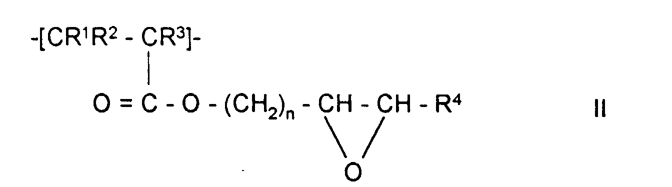 worin
R¹, R² und R³ unabhängig voneinander H oder CH₃,
R⁴ H, Alkyl mit 1-5 C-Atomen oder Aryl mit 6-12 C-Atomen
und
n eine ganzen Zahl zwischen 1 und 5
bedeuten, enthält.

5. Polymeres Sorbens nach Anspruch 1 oder 2, dadurch gekennzeichnet, daß das aufpolymerisierte Polymer Monomereinheiten der Formel V, 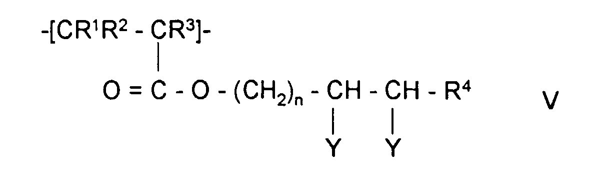 worin
R¹, R² und R³ unabhängig voneinander H oder CH₃,
R⁴ H, Alkyl mit 1-5 C-Atomen oder Aryl mit 6-12 C-Atomen
n eine ganzen Zahl zwischen 1 und 5,
ein Rest Y einen Rest der Formel VI und der andere Rest Y OH 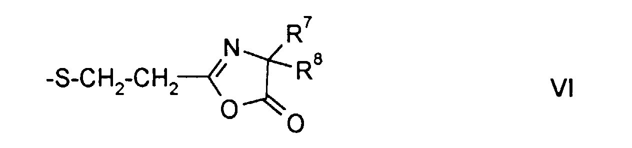 und
R⁷ und R⁸ unabhängig voneinander H oder Alkyl mit 1-5 C-Atomen
bedeuten.

6. Polymeres Sorbens nach Anspruch 1 oder 2, dadurch gekennzeichnet, daß das aufpolymerisierte Polymer Monomereinheiten der Formel III, 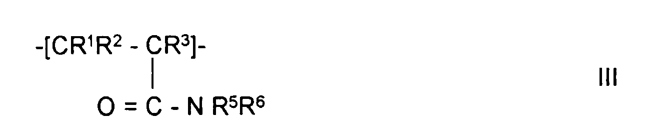 worin
R¹, R² und R³ unabhängig voneinander H oder CH₃,
R⁵ H, mit -COOH, mit -SO₃H oder mit -NR⁷R⁸ substituiertes Alkyl mit 1-5 C-Atomen oder mit -COOH, -SO₃H oder mit -NR⁷R⁸ substituiertes Aryl mit 6-12 C-Atomen,
R⁶ mit -COOH, mit mit -SO₃H oder mit -NR⁷R⁸ substituiertes Alkyl mit 1-5 C-Atomen oder mit -COOH, -SO₃H oder mit -NR⁷R⁸ substituiertes Aryl mit 6-12 C-tomen, wobei R⁵ und R⁶ so abgestimmt sind, daß entweder beide Reste sauer oder basisch oder einer der Reste neutral ist,
und
R⁷ und R⁸ unabhängig voneinander H, Alkyl mit 1-5 C-Atomen
bedeuten, enthält.

7. Polymeres Sorbens nach Anspruch 1 oder 2, dadurch gekennzeichnet, daß das aufpolymerisierte Polymer Monomereinheiten der Formel IV, 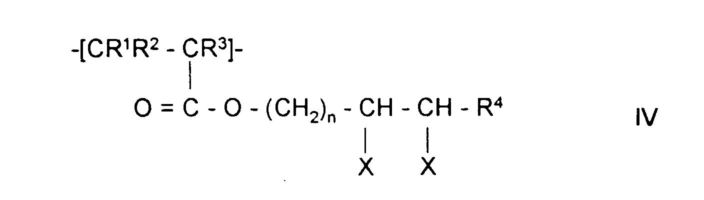 worin
R¹, R² und R³ unabhängig voneinander H oder CH₃,
R⁴ H, Alkyl mit 1-5 C-Atomen oder Aryl mit 6-12 C-Atomen
n eine ganzen Zahl zwischen 1 und 5,
ein Rest X einen Separationseffektor, sowie der andere Rest X OH bedeuten, enthält.

8. Polymeres Sorbens nach Anspruch 7, dadurch gekennzeichnet, daß der Separationseffektor eine ionische Gruppe, ausgewählt aus -PO₄H₂, -SO₃H, -NR⁷R⁸ oder -N⁺R⁷R⁸R⁹ darstellt, worin
R⁷ und R⁸ unabhängig voneinander H, Alkyl mit 1-5 C-Atomen
und
R⁹ Alkyl mit 1-5 C-Atomen
mit der Maßgabe, daß wenn X = -N⁺R⁷R⁸R⁹, R⁷ und R⁸ nicht H sein können, bedeutet.

9. Polymeres Sorbens nach Anspruch 7, dadurch gekennzeichnet, daß der Separationseffektor eine hydrophobe Gruppierung -OR¹⁰ oder -NHR¹⁰, wobei
R¹⁰ C₁-C₂₀-Alkyl, C₆-C₂₅-Aryl, C₇-C₂₅-Alkylaryl oder C₇-C₂₅-Arylalkyl bedeuten, und wobei diese Reste auch mit Nitril oder C₁-C₅-Alkoxy derivatisiert sein können, und wobei auch eine oder mehrere nicht benachbarte CH₂-Gruppen durch NH oder O oder auch eine oder mehrere CH Gruppen durch N ersetzt sein können,
bedeutet.

10. Polymeres Sorbens nach Anspruch 7, dadurch gekennzeichnet, daß der Separationseffektor eine Metallchelat-Affinitätsgruppe darstellt.

11. Polymeres Sorbens nach Anspruch 7, dadurch gekennzeichnet, daß der Separationseffektor einen thiophilen Rest darstellt.

12. Affinitätsträger herstellbar aus einem polymeren Sorbens nach einem der Ansprüche 1, 2, 3, 4, 5, 6 oder 8.

13. Immobilisiertes Enzym herstellbar aus einem polymeren Sorbens nach einem der Ansprüche 1, 2, 3, 4, 5, 6 oder 8.

14. Formkörper im wesentlichen bestehend aus einem polymeren Sorbens nach einem der Ansprüche 1 bis 11.

15. Formkörper, an dem ein Affinitätsligand oder ein Enzym immobilisiert ist, herstellbar aus einem polymeren Sorbens nach einem der Ansprüche 1, 2, 3, 4, 5, 6 oder 8.

16. Verfahren zur chromatographischen Stofftrennung, dadurch gekennzeichnet, daß ein polymeres Sorbens nach einem der Ansprüche 1 - 12 verwendet wird.

## Claims

1. Polymeric sorbent obtainable by the following reaction steps:
a) introducing polymerizing double bonds by reacting a polyamide with a compound which comprises both a polymerizable double bond and also an oxirane ring;
b) polymerizing monomers onto the polyamide derivatized in accordance with step a), the said monomers comprising an epoxide group, an azlactone group or a precursor group for an azlactone group or a separation effector;
c) optionally converting the said epoxide or azlactone group into a separation effector.

2. Polymeric sorbent according to Claim 1, where in step a) the compound used which comprises both a polymerizable double bond and also an o irane ring is 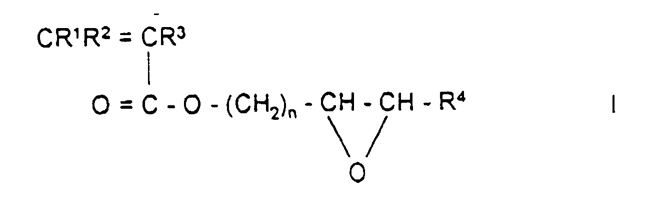 where
R¹, R² and R³ independently of one another are H or CH₃,
R⁴ is H, alkyl with from 1 to 5 carbon atoms or aryl with from 6 to 12 carbon atoms
and
n is an integer from 1 to 5.

3. Polymeric sorbent according to Claim 1 or 2, characterized in that the monomer used in reaction step b) is acrylic acid.

4. Polymeric sorbent according to Claim 1 or 2, characterized in that the polymer which is the graft comprises monomer units of the formula II 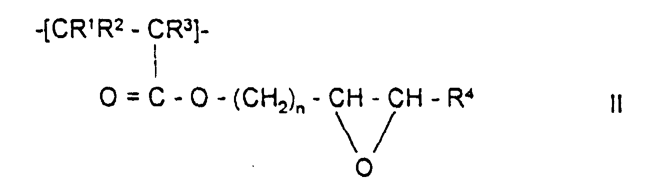 where
R¹, R² and R³ independently of one another are H or CH₃,
R⁴ is H, alkyl with from 1 to 5 carbon atoms or aryl with from 6 to 12 carbon atoms
and
n is an integer from 1 to 5.

5. Polymeric sorbent according to Claim I or 2, characterized in that the polymer which is the graft comprises monomer units of the formula V 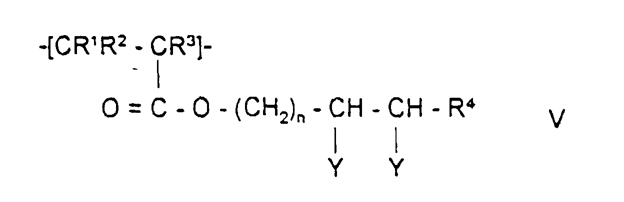 where
R¹, R² and R³ independently of one another are H or CH₃,
R⁴ is H, alkyl with from 1 to 5 carbon atoms or aryl with from 6 to 12 carbon atoms,
n is an integer from 1 to 5,
one radical Y is a radical of the formula VI and the other radical Y is OH 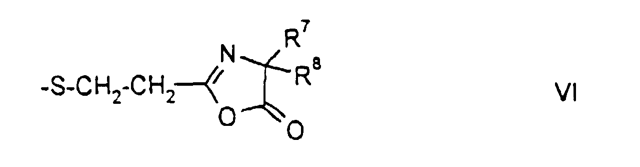 and
R⁷ and R⁸ independently of one another are H or alkyl with from 1 to 5 carbon atoms.

6. Polymeric sorbent according to Claim 1 or 2, characterized in that the polymer which is the graft comprises monomer units of the formula III 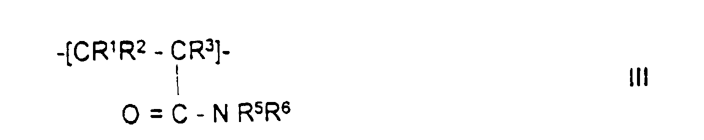 where
R¹, R² and R³ independently of one another are H or CH₃,
R⁵ is H, or alkyl with from 1 to 5 carbon atoms substituted with -COOH, with -SO₃H or with NR⁷R⁸, or aryl with from 6 to 12 carbon atoms and substituted with -COOH, -SO₃H or with -NR⁷R⁸,
R⁶ is alkyl with from 1 to 5 carbon atoms substituted with -COOH, with -S03H or with NR⁷R⁸, or aryl with from 6 to 12 carbon atoms and substituted with -COOH, -SO₃H or -NR⁷R⁸, where R⁵ and R⁶ are selected so that either both radicals are acid or basic or one of the radicals is neutral,
and
R⁷ and R⁸ independently of one another are H or alkyl with from 1 to 5 carbon atoms.

7. Polymeric sorbent according to Claim 1 or 2, characterized in that the polymer which is the graft comprises monomer units of the formula IV 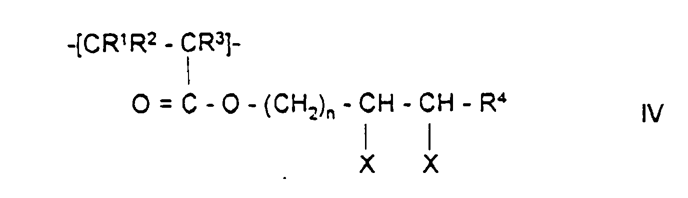 where
R¹, R² and R³ independently of one another are H or CH₃,
R⁴ is H, alkyl with from 1 to 5 carbon atoms or aryl with from 6 to 12 carbon atoms,
n is an integer from 1 to 5,
one radical X is a separation effector and the other radical X is OH.

8. Polymeric sorbent according to Claim 7, characterized in that the separation effector is an ionic radical selected from the group consisting of -PO₄H₂, -SO₃H, -NR⁷R⁸ and -N⁺R⁷R⁸R⁹, where
R⁷ and R⁸ independently of one another are H or alkyl with from 1 to 5 carbon atoms
and
R⁹ is alkyl with from 1 to 5 carbon atoms
with the proviso that if X = -N⁺R⁷R⁸R⁹, R⁷ and R⁸ may not be H.

9. Polymeric sorbent according to Claim 7, characterized in that the separation effector is a hydrophobic moiety -OR¹⁰ or -NHR¹⁰, where
R¹⁰ is C₁-C₂₀-alkyl, C₆-C₂₅-aryl, C₇-C₂₅-alkylaryl or C₇-C₂₅-arylalkyl, and where these radicals may also be derivatized with nitrile or C₁-C₅-alkoxy, and where, furthermore, one or more non-adjacent CH₂ radicals may be replaced by NH or O or else one or more CH radicals may be replaced by N.

10. Polymeric sorbent according to Claim 7, characterized in that the separation effector is a metal chelate affinity group.

11. Polymeric sorbent according to Claim 7, characterized in that the separation effector is a thiophilic radical.

12. Affinity support which can be prepared from a polymeric sorbent according to one of Claims 1, 2, 3, 4, 5, 6 or 8.

13. Immobilized enzyme which can be prepared from a polymeric sorbent according to one of Claims 1, 2, 3, 4, 5, 6 or 8.

14. Shaped article consisting essentially of a polymeric sorbent according to one of claims 1 to 11.

15. Shaped article on which an affinity ligand or an enzyme is immobilized, and which can be prepared from a polymeric sorbent according to one of Claims 1, 2, 3, 4, 5, 6 or 8.

16. Process for the chromatographic separation of substances, characterized in that a polymeric sorbent according to one of claims 1 to 12 is used.

## Revendications

1. Sorbant polymère obtenu au moyen des étapes réactionnelles suivantes :
a) introduction de doubles liaisons polymérisables par réaction d'un polyamide avec un composé contenant aussi bien une double liaison polymérisable qu'un cycle oxirane,
b) polymérisation de monomères sur le polyamide dérivé selon l'étape a), lesdits monomères contenant un groupe époxyde, un groupe azolactone ou un groupe précurseur d'un groupe azolactone ou un effecteur de séparation,
c) transformation optionnelle du groupe époxyde ou azolactone précité en un effecteur de séparation.

2. Sorbant polymère selon la revendication 1, caractérisé en ce que l'on utilise dans l'étape a), comme composé contenant aussi bien une double liaison polymérisable qu'un cycle oxirane, un composé de formule I 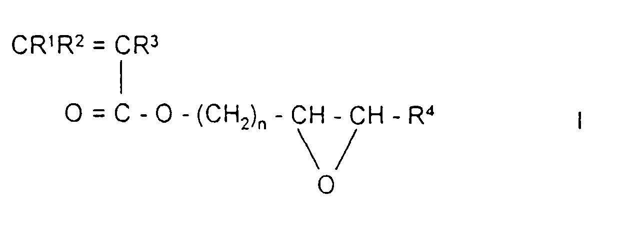 où
R¹, R² et R³ représentent, indépendamment les uns des autres, H ou CH₃,
R⁴ représente H, un alkyle comportant 1 à 5 atomes de C ou un aryle comportant 6 à 12 atomes de C
et
n est un nombre entier compris entre 1 et 5.

3. Sorbant polymère selon la revendication 1 ou 2, caractérisé en ce que l'on utilise comme monomère dans l'étape réactionnelle b) l'acide acrylique.

4. Sorbant polymère selon la revendication 1 ou 2, caractérisé en ce que le polymère greffé contient des unités monomères de formule II 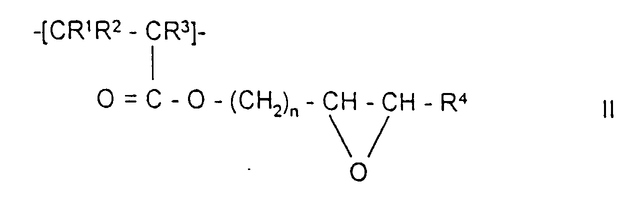 où
R¹, R² et R³ représentent, indépendamment les uns des autres, H ou CH₃,
R⁴ représente H, un alkyle comportant 1 à 5 atomes de C ou un aryle comportant 6 à 12 atomes de C
et
n est un nombre entier compris entre 1 et 5.

5. Sorbant polymère selon la revendication 1 ou 2, caractérisé en ce que le polymère greffé contient des unités monomères de formule V 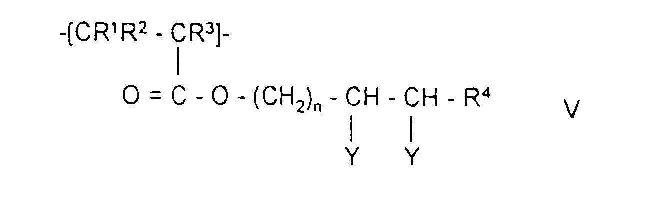
R¹, R² et R³ représentent, indépendamment les uns des autres, H ou CH₃,
R⁴ représente H, un alkyle comportant 1 à 5 atomes de C ou un aryle comportant 6 à 12 atomes de C
et
n est un nombre entier compris entre 1 et 5,
l'un des restes Y représente un reste de formule VI et l'autre reste Y représente OH 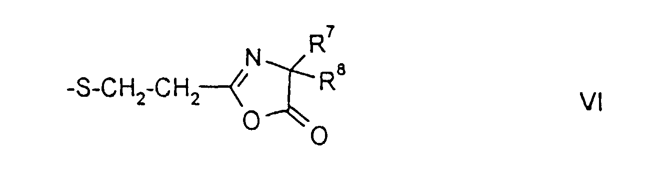 et
R⁷ et R⁸ représentent, indépendamment l'un de l'autre, H ou un alkyle comportant 1 à 5 atomes de C.

6. Sorbant polymère selon la revendication 1 ou 2, caractérisé en ce que le polymère greffé contient des unités monomères de formule III 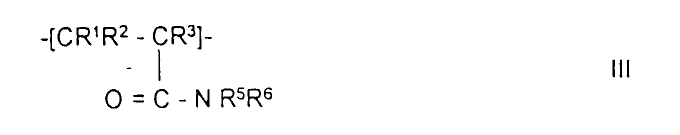 où
R¹, R² et R³ représentent, indépendamment les uns des autres, H ou CH₃,
R⁵ représente H, un alkyle comportant 1 à 5 atomes de C substitué par -COOH, -SO₃H ou -NR⁷R⁸ ou un aryle comportant 6 à 12 atomes de C substitué par -COOH, -SO₃H ou -NR⁷R⁸,
R⁶ un alkyle comportant 1 à 5 atomes de C substitué par -COOH, -SO₃H ou -NR⁷R⁸ ou un aryle comportant 6 à 12 atomes de C substitué par -COOH, -SO₃H ou -NR⁷R⁸, R⁵ et R⁶ étant choisis de telle manière que soit les deux restes sont acides ou basiques, soit l'un des restes est neutre
et
R⁷ et R⁸ représentent, indépendamment l'un de l'autre, H, un alkyle comportant 1 à 5 atomes de C.

7. Sorbant polymère selon la revendication 1 ou 2, caractérisé en ce que le polymère greffé contient des unités monomères de formule IV 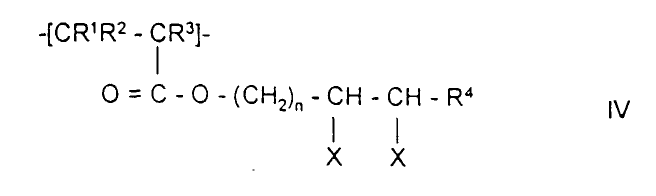 où
R¹, R² et R³ représentent, indépendamment les uns des autres, H ou CH₃,
R⁴ représente H, un alkyle comportant 1 à 5 atomes de C ou un aryle comportant 6 à 12 atomes de C,
n est un nombre entier compris entre 1 et 5,
l'un des restes X représente un effecteur de séparation et l'autre reste X représente OH.

8. Sorbant polymère selon la revendication 7, caractérisé en ce que l'effecteur de séparation est un groupe ionique choisi parmi -PO₄H₂, -SO₃H, -NR⁷R⁸ ou -N⁺R⁷R⁸R⁹ où
R⁷ et R⁸ représentent, indépendamment l'un de l'autre,
H, un alkyle comportant 1 à 5 atomes de C
et
R⁹ représente un alkyle comportant 1 à 5 atomes de C, sous réserve que lorsque X = -N⁺R⁷R⁸R⁹, R⁷ et R⁸ ne peuvent être H.

9. Sorbant polymère selon la revendication 7, caractérisé en ce que l'effecteur de séparation est un groupement hydrophobe -OR¹⁰ ou -NHR¹⁰ où
R¹⁰ représente un alkyle en C₁-C₂₀, un aryle en C₆-C₂₅, un alkylaryle en C₇-C₂₅ ou un arylalkyle en C7-C25, ces restes pouvant être également substitués par un nitrile ou un alcoxy en C₁-C₅ et un ou plusieurs groupes CH₂ non adjacents pouvant être également remplacés par NH ou O ou un ou plusieurs groupes CH pouvant être également remplacés par N.

10. Sorbant polymère selon la revendication 7, caractérisé en ce que l'effecteur de séparation est un groupe d'affinité chélate métallique.

11. Sorbant polymère selon la revendication 7, caractérisé en ce que l'effecteur de séparation est un reste thiophilique.

12. Support d'affinité pouvant être préparé à partir d'un sorbant polymère selon l'une des revendications 1, 2, 3, 4, 5, 6 ou 8.

13. Enzyme immobilisée pouvant être préparée à partir d'un sorbant polymère selon l'une des revendications 1, 2, 3, 4, 5, 6 ou 8.

14. Corps moulé essentiellement constitué d'un sorbant polymère selon l'une des revendications 1 à 11.

15. Corps moulé sur lequel est immobilisé un ligand d'affinité ou une enzyme, pouvant être obtenu à partir d'un sorbant polymère selon l'une des revendications 1, 2, 3, 4, 5, 6 ou 8.

16. Procédé pour la préparation de substances par chromatographie, caractérisé en ce que l'on utilise un sorbant polymère selon l'une des revendications 1 à 12.
